(19) Europäisches Patentamt / European Patent Office / Office européen des brevets

(11) **EP 4 098 993 A1**

(12) **EUROPEAN PATENT APPLICATION**

(43) Date of publication:
**07.12.2022 Bulletin 2022/49**

(21) Application number: **22176417.8**

(22) Date of filing: **31.05.2022**

(51) International Patent Classification (IPC):
**G01N 1/20** (2006.01)   **G01N 33/18** (2006.01)
**G01N 1/10** (2006.01)

(52) Cooperative Patent Classification (CPC):
**G01N 1/2035; G01N 33/18; G01N 33/1813;
G01N 33/182;** G01N 2001/1031; G01N 2001/205

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.05.2021 JP 2021090822**

(71) Applicant: **NGK Spark Plug Co., Ltd.
Nagoya-shi, Aichi 461-0005 (JP)**

(72) Inventor: **Mihara, Shunya
Aichi (JP)**

(74) Representative: **Grünecker Patent- und
Rechtsanwälte
PartG mbB
Leopoldstraße 4
80802 München (DE)**

(54) **LIQUID QUALITY MEASUREMENT APPARATUS AND LIQUID QUALITY MEASUREMENT SYSTEM**

(57)     An object is to appropriately add a liquid different from a measurement liquid to the measurement liquid within a circulation path.

It is provided a liquid quality measurement apparatus 1 includes a piping 90 which constitutes a circulation path 10 through which a measurement liquid S flows, and a liquid quality sensor 31 for measuring the quality of the measurement liquid S within the circulation path 10. The liquid quality measurement apparatus 1 further includes an addition tank 33 which is connected to the piping 90 via an inlet port 54 and an outlet port 55, constitutes a portion of the circulation path 10, and has an internal space 50. The addition tank 33 has an introduction portion 70 through which a liquid different from the measurement liquid S is introduced into the internal space 50 from the outside of the addition tank 33, and a ventilation portion 60 through which air is released from the internal space 50 to the outside. An addition tank internal channel 56 is formed at a bottom portion of the internal space 50. The addition tank internal channel 56 connects the inlet port 54 and the outlet port 55. The measurement liquid S flows through the addition tank internal channel 56. The introduction portion 70 and the ventilation portion 60 are disposed in a wall surface of the addition tank 33 that faces the internal space 50 above the addition tank internal channel 56.

Fig. 3

EP 4 098 993 A1

## Description

The present invention relates to a liquid quality measurement apparatus and to a liquid quality measurement system.

Patent Document 1 discloses a hot water quality measurement apparatus including a water quality sensor. In this hot water quality measurement apparatus, the quality of hot water flowing through a main piping is measured by the water quality sensor.

[Patent Document 1] Japanese Patent Application Laid-Open (kokai) No. 2012-83293

The technique of Patent Document 1 may be utilized in such a manner as to feed a measurement liquid (liquid whose quality is to be measured) to the main piping and measure the quality of the measurement liquid by the liquid quality sensor. However, in this case, the measurement liquid is continuously fed to the main piping during the measurement, and therefore, the consumption amount of the measurement liquid increases. A conceivable way to overcome such a problem is to measure the quality of the measurement liquid in a circulation path described in Patent Document 1, with the measurement liquid being circulated through the circulation path. However, in the case where a liquid different from the measurement liquid is added to the measurement liquid within the circulation path and then the quality of the measurement liquid is measured, since the interior of the circulation path is a closed space, there is a possibility that the different liquid cannot be added appropriately, or the measurement liquid flows reversely into a supply line for the different liquid, thereby contaminating the supply line.

The present invention provides a technique capable of appropriately adding a liquid different from a measurement liquid to the measurement liquid within a circulation path.

[Means for Solving the Problems]

[1] A liquid quality measurement apparatus of the present invention includes a piping that constitutes a circulation path through which a measurement liquid flows, and a liquid quality sensor for measuring a quality of the measurement liquid within the circulation path. The liquid quality measurement apparatus of the present invention further includes an addition tank that is connected to the piping via an inlet port and an outlet port, constitutes a portion of the circulation path, and has an internal space. The addition tank has an introduction portion through which a liquid different from the measurement liquid is introduced into the internal space from outside of the addition tank, and a ventilation portion through which air is released from the internal space to the outside. An addition tank internal channel is formed at a bottom portion of the internal space, the addition tank internal channel connecting the inlet port and the outlet port and allowing the measurement liquid to flow through the addition tank internal channel. The introduction portion and the ventilation portion are disposed in a wall surface of the addition tank that faces the internal space above the addition tank internal channel.

This liquid quality measurement apparatus includes an addition tank that constitutes a portion of the circulation path, and an introduction portion and a ventilation portion are provided in the addition tank. Therefore, this liquid quality measurement apparatus can appropriately add a liquid different from the measurement liquid to the internal space of the addition tank through the introduction portion while releasing air from the internal space of the addition tank through the ventilation portion.

[2] A maximum width Wmax of the addition tank internal channel may be determined to satisfy a condition of the following inequality (1) in a relation between the maximum width Wmax and a diameter R1 of the inlet port, and satisfy a condition of the following inequality (2) in a relation between the maximum width Wmax and a diameter R2 of the outlet port.

$$R1 \times 0.9 < Wmax < R1 \times 1.1 \qquad (1)$$

$$R2 \times 0.9 < Wmax < R2 \times 1.1 \qquad (2)$$

This configuration can reduce the amount of the measurement liquid necessary to be charged into the circulation path.

[3] In the addition tank, a distance in a height direction between a lowest position of the addition tank internal channel and a lowest position of the inlet port may be less than 0.1 times the diameter R1 of the inlet port, and a distance in the height direction between the lowest position of the addition tank internal channel and a lowest position of the outlet port may be less than 0.1 times the diameter R2 of the outlet port.

By virtue of this configuration, when the measurement liquid within the circulation path is drained, it is possible to prevent the measurement liquid from remaining at the bottom of the addition tank internal channel.

[4] An upper end of the addition tank internal channel may be located higher than an upper end of the inlet port and an upper end of the outlet port.

This configuration can reduce the amount of the measurement liquid necessary to be charged into the circulation path.

[5] The addition tank may have a sloping surface that extends from the upper end of the addition tank internal channel and slopes upward in a direction away from the addition tank internal channel.

By virtue of this configuration, the measurement liquid having accumulated in the internal space of the addition tank is guided to the addition tank internal channel by the sloping surface, and thus, the measurement liquid is easily drained from the addition tank internal channel.

[6] The introduction portion for introducing the different liquid may be disposed right above the addition tank internal channel.

This configuration can prevent the different liquid introduced from the introduction portion from being uselessly supplied to a location outside the addition tank internal channel.

[7] The introduction portion may be provided on a top wall of the addition tank.

By virtue of this configuration, flow of the measurement liquid into the introduction portion can be easily prevented.

[8] An opening of the ventilation portion may have a sectional area greater than a sectional area of the inlet port and a sectional area of the outlet port.

By virtue of this configuration, an amount of air that is equal to or greater than the amount of the measurement liquid supplied to the internal space can be easily released from the ventilation portion. Accordingly, flow of the measurement liquid into the introduction portion can be easily prevented.

[9] The ventilation portion may be provided in a side wall of the addition tank.

By virtue of this configuration, when the level of the measurement liquid exceeds a predetermined level in the internal space of the addition tank, an excessive portion of the measurement liquid can be drained to the outside.

[10] A liquid quality measurement system of the present invention includes a liquid quality measurement apparatus set forth in any one of the above [1] to [9], an adjustment liquid supply section, and a monitoring apparatus. The adjustment liquid supply section supplies an adjustment liquid, as a different liquid, through the introduction portion under control performed by the liquid quality measurement apparatus. The monitoring apparatus monitors the quality of the measurement liquid measured by the liquid quality sensor. The liquid quality measurement apparatus starts liquid quality measurement operation by the liquid quality sensor after liquid quality adjustment performed for the measurement liquid by using the adjustment liquid has been completed, or adopts a measurement value measured by the liquid quality sensor after completion of the liquid quality adjustment as a liquid quality value of the measurement liquid.

By virtue of this configuration, the liquid quality value of the measurement liquid in a state after completion of the liquid quality adjustment can be obtained.

[11] The liquid quality measurement apparatus may further include a pH sensor for measuring pH of the measurement liquid, and, as the liquid quality adjustment, the liquid quality measurement apparatus may cause the adjustment liquid supply section to supply the adjustment liquid until the liquid quality measurement apparatus determines that the pH has exceeded a threshold value.

By virtue of this configuration, the liquid quality value of the measurement liquid whose pH has been adjusted can be obtained.

According to the present invention, a liquid different from the measurement liquid can be appropriately added to the measurement liquid within the circulation path.

[Fig. 1] Fig. 1 is a block diagram schematically showing a liquid quality measurement system.

[Fig. 2] Fig. 2 is a plan view of an addition tank.

[Fig. 3] Fig. 3 is a side sectional view of the addition tank.

[Fig. 4] Fig. 4 is a horizontal sectional view of the addition tank.

[Fig. 5] Fig. 5 is a cross-sectional view of the addition tank.

[Fig. 6] Fig. 6 is a flowchart illustrating the flow of operation of a liquid quality measurement apparatus.

[Modes for Carrying out the Invention]

1. First embodiment

1-1. Configuration of liquid quality measurement system 100

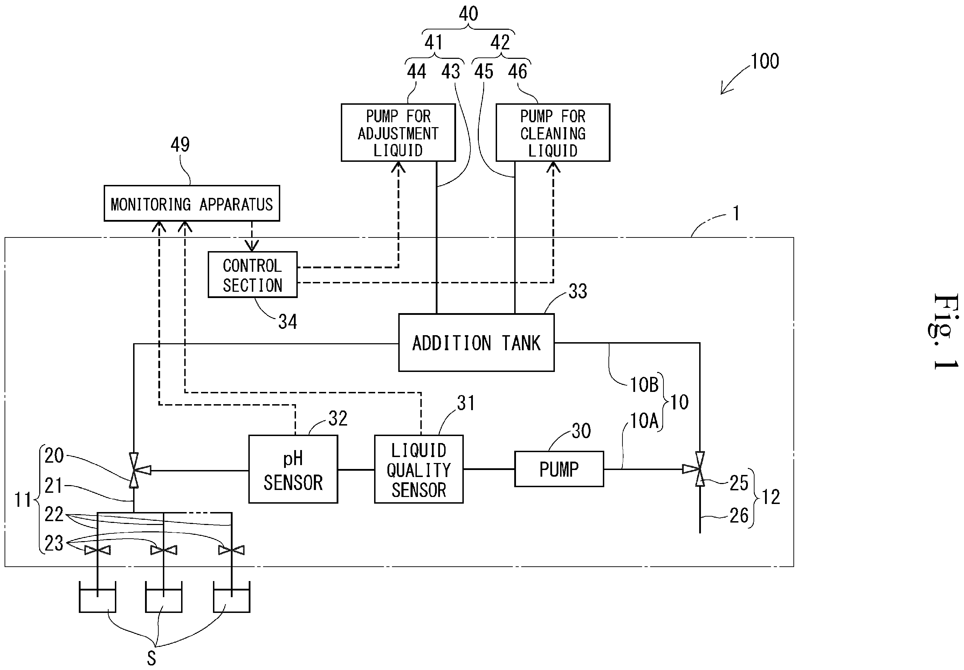

A liquid quality measurement system 100 shown in Fig. 1 is a system for measuring the qualities of measurement liquids S and is mounted on, for example, a liquid piping line of a material tank of a biogas plant. The liquid quality measurement system 100 includes a liquid quality measurement apparatus 1. The liquid quality measurement apparatus 1 includes a circulation path 10 through which each measurement liquid S is circulated, a measurement liquid supply section 11 for supplying the measurement liquid S to the circulation path 10, and a measurement liquid draining section 12 for draining the measurement liquid S from the circulation path 10.

The measurement liquid supply section 11 includes a three-way valve 20 provided in the circulation path 10, a common path 21 connected to the three-way valve 20, a plurality of branch paths 22 branching from the common path 21, and two-way valves 23 provided in the branch paths 22. The three-way valve 20 is switched between a permission state in which the three-way valve 20 permits supply of each measurement liquid S from the common path 21 to the circulation path 10 and a prohibition state in which the three-way valve 20 prohibits the supply. The branch paths 22 are provided for the respective measurement liquids S and suck the corresponding measurement liquids S. Each two-way valve 23 is switched between an open state for opening the corresponding branch path 22 and a closed state for closing the corresponding branch path 22. The measurement liquid supply section 11 supplies each measurement liquid S to the circulation path 10 by switching the three-way valve 20 to the permission state and switching the corresponding two-way valve 23 to the open state.

The measurement liquid draining section 12 includes a three-way valve 25 provided in the circulation path 10, a drain passage 26 connected to the three-way valve 25. The three-way valve 25 is switched between a permission state in which the three-way valve 25 permits drainage of the measurement liquid S from the circulation path 10 to the drain passage 26 and a prohibition state in which the three-way valve 25 prohibits the drainage. The measurement liquid draining section 12 drains the measurement liquid S within the circulation path 10 by switching the three-way valve 25 to the permission state.

The circulation path 10 includes a first path 10A and a second path 10B connected to the three-way valve 20 and the three-way valve 25, respectively. The three-way valve 20 and the three-way valve 25 are switched among their first states for permitting inflow of each measurement liquid S from the common path 21 of the measurement liquid supply section 11 to the circulation path 10 (more specifically, to the first path 10A), their second states in which the circulation path 10 forms a closed path, and their third states for permitting drainage of the measurement liquid S from the circulation path 10 (more specifically, from the first path 10A) to the drain passage 26 of the measurement liquid draining section 12. Notably, when the three-way valve 20 and the three-way valve 25 are in their first states, drainage of the measurement liquid S from the circulation path 10 is prohibited. When the three-way valve 20 and the three-way valve 25 are in their second states, the inflow of the measurement liquid S to the circulation path 10 and the drainage of the measurement liquid S from the circulation path 10 are prohibited. When the three-way valve 20 and the three-way valve 25 are in their third states, the inflow of the measurement liquid S to the circulation path 10 is prohibited.

The liquid quality measurement apparatus 1 includes a pump 30, a liquid quality sensor 31, a pH sensor 32, an addition tank 33, and a control section 34.

The pump 30 is provided in the circulation path 10 (more specifically, the first path 10A). In the case where the three-way valve 20 and the three-way valve 25 are in their first states, the pump 30 sucks the measurement liquid S, supplied from the measurement liquid supply section 11, into the circulation path 10. In the case where the three-way valve 20 and the three-way valve 25 are in their second states, the pump 30 circulates the measurement liquid S within the circulation path 10. In the case where the three-way valve 20 and the three-way valve 25 are in their third states, the pump 30 drains the measurement liquid S within the circulation path 10 from the measurement liquid draining section 12.

The liquid quality sensor 31 is a sensor for measuring the quality (more specifically, a property different from pH) of the measurement liquid S. The liquid quality sensor 31 is an optical sensor for measuring the concentration of a specific ion (ammonium ion in the present embodiment) contained in the measurement liquid S. The liquid quality sensor 31 is provided in the circulation path 10 (more specifically, the first path 10A) and measures the quality of the measurement liquid S within the circulation path 10. The liquid quality sensor 31 outputs a measurement value. The measurement value output from the liquid quality sensor 31 is input to a monitoring apparatus 49 and input to the control section 34 via the monitoring apparatus 49. Notably, the liquid quality sensor 31 is not limited to an optical sensor and may be any of sensors operating in accordance with other measurement schemes, for example, a membrane-type sensor. The measurement target of the liquid quality sensor 31 is not limited to ammonium ion and may be other ions such as phosphate ion and metal ion or ORP (oxidation-reduction potential).

The pH sensor 32 is a sensor for measuring the pH of the measurement liquid S. The pH sensor 32 is provided in the circulation path 10 (more specifically, the first path 10A) and measures the pH of the measurement liquid S within the circulation path 10. The pH sensor 32 outputs a measurement value. The measurement value output from the pH sensor 32 is input to the monitoring apparatus 49 and input to the control section 34 via the monitoring apparatus 49.

The addition tank 33 constitutes a portion of the circulation path 10 (more specifically, the second path 10B). The addition tank 33 will be described in detail later.

The control section 34 is, for example, a MCU (Micro Controller Unit) and includes a CPU, a ROM, a RAM, etc. The control section 34 controls operations of various devices of the liquid quality measurement system 100 (specifically, the measurement liquid supply section 11, the measurement liquid draining section 12, the pump 30, the liquid quality sensor 31, the pH sensor 32, and a supply section 40).

The liquid quality measurement system 100 includes the supply section 40 and the monitoring apparatus 49.

The supply section 40 includes an adjustment liquid supply section 41 and a cleaning liquid supply section 42. The adjustment liquid supply section 41 is connected to the addition tank 33 and supplies an adjustment liquid to the addition tank 33. The adjustment liquid supply section 41 includes an adjustment liquid supply passage 43 connected to the addition tank 33 and a pump 44 for adjustment liquid provided in the adjustment liquid supply passage 43. The adjustment liquid supply section 41 supplies the adjustment liquid to the addition tank 33 via the adjustment liquid supply passage 43. In the present embodiment, the adjustment liquid is a pH adjustment liquid such as acid, alkali, etc. The cleaning liquid supply section 42 is connected to the addition tank 33 and supplies a cleaning liquid to the addition tank 33. The cleaning liquid supply section 42 includes a cleaning liquid supply passage 45 connected to the addition tank 33 and a pump 46 for cleaning liquid provided in the cleaning liquid supply passage 45. The cleaning liquid supply section 42 supplies the cleaning liquid to the addition tank 33 via the cleaning liquid supply passage 45. Notably, the adjustment liquid is not limited to a pH control agent and may be, for example, a chemical solution for color reaction.

The monitoring apparatus 49 monitors the results of the measurements by the liquid quality sensor 31 and the pH sensor 32. Namely, the monitoring apparatus 49 monitors the pH and ammonium ion concentration of the measurement liquid S.

1-2. Structure of addition tank 33

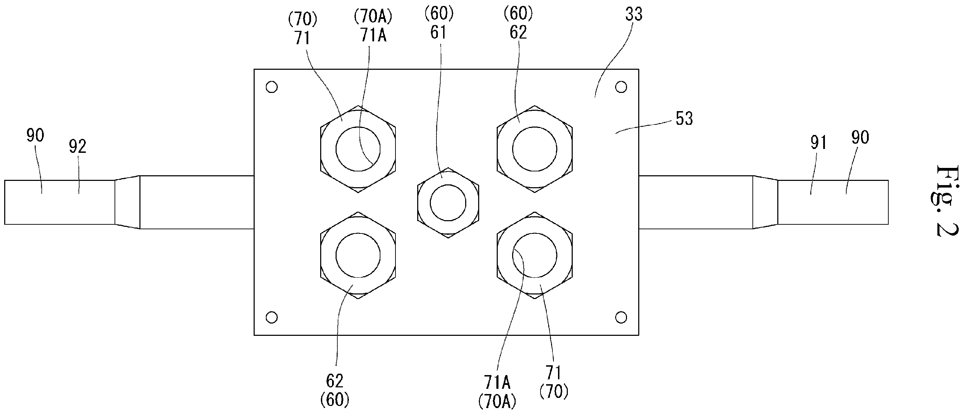

As described above, the addition tank 33 constitutes a portion of the circulation path 10. As shown in Figs. 2 to 5, the addition tank 33 has a box-like shape and has an internal space 50. The addition tank 33 has a bottom wall 51, a side wall 52 rising from the bottom wall 51 and surrounding the four sides of the internal space 50, and a top wall 53. The internal space 50 is surrounded by the bottom wall 51, the side wall 52, and the top wall 53.

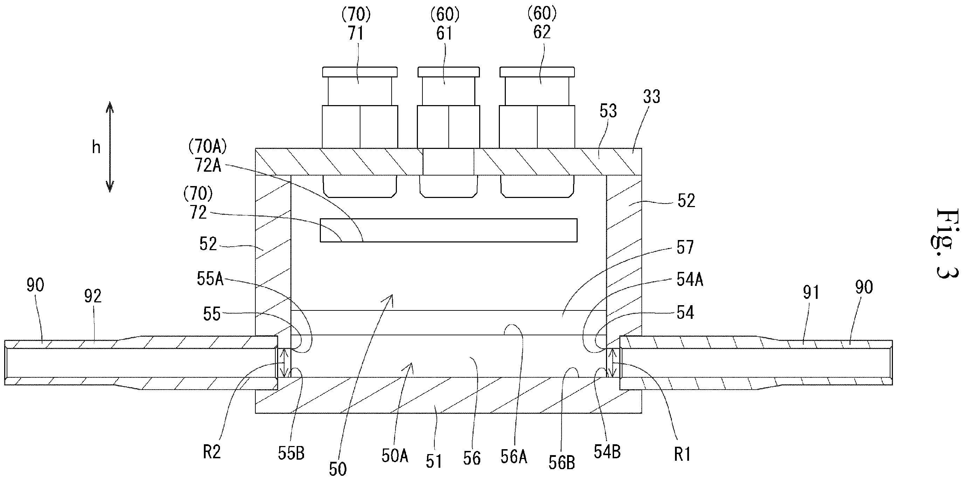

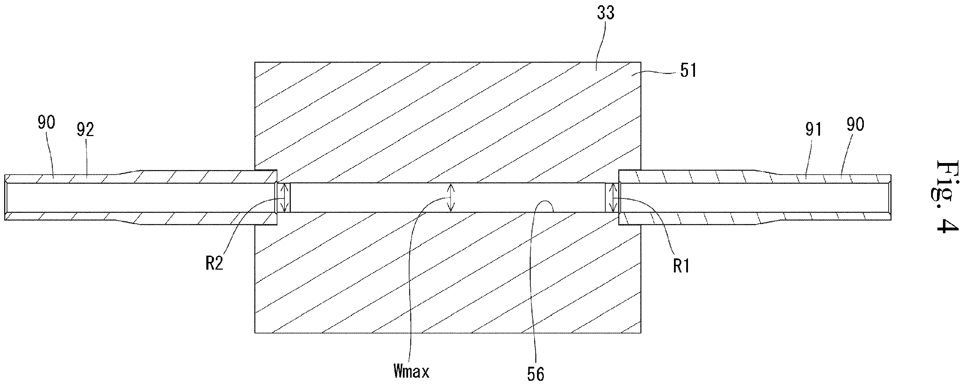

As shown in Figs. 3 and 4, the liquid quality measurement apparatus 1 includes a piping 90 which constitutes the circulation path 10. The piping 90 includes a first pipe 91 and a second pipe 92. An inlet port 54 and an outlet port 55 are formed in the side wall 52 of the addition tank 33. Both the sectional shapes of the inlet port 54 and the outlet port 55 are circular. The addition tank 33 is connected to the first pipe 91 via the inlet port 54 and is connected to the second pipe 92 via the outlet port 55.

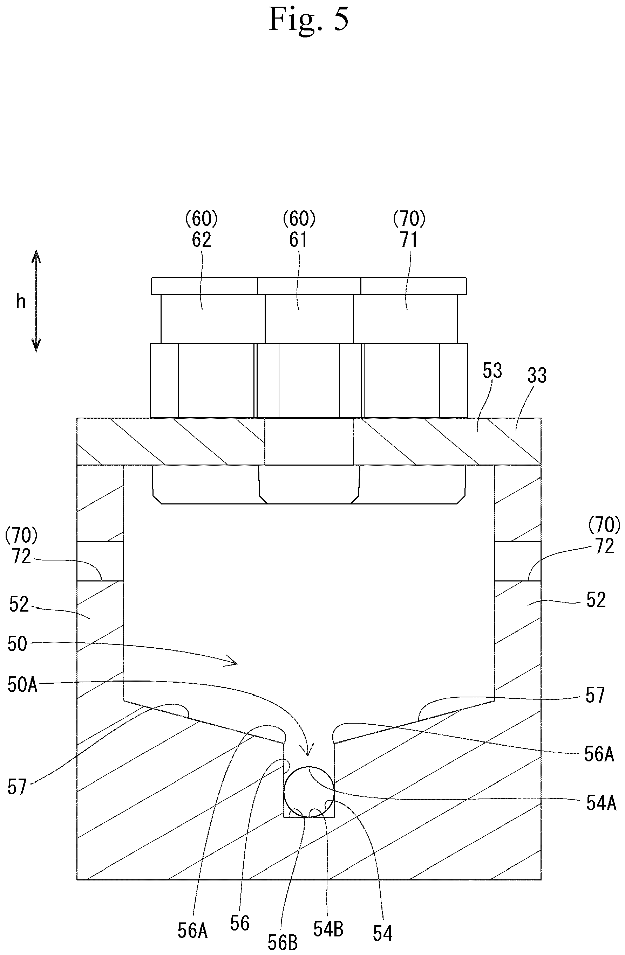

As shown in Figs. 3 and 5, an addition tank internal channel 56 is formed in a bottom portion 50A of the internal space 50. The addition tank internal channel 56 connects the inlet port 54 and the outlet port 55, and the measurement liquid S flows through the addition tank internal channel 56. The addition tank internal channel 56, which has the shape of a groove which is open upward, extends straight. The bottom surface of the addition tank internal channel 56 is a surface that is flat along a horizontal direction. The side surfaces of the addition tank internal channel 56 are flat surfaces orthogonal to the bottom surface.

As shown in Fig. 5, the width of the addition tank internal channel 56 (the distance between the opposite side surfaces of the addition tank internal channel 56) is constant in the vertical direction. As shown in Fig. 4, the maximum width Wmax of the addition tank internal channel 56 preferably satisfies the condition of the following inequality (1) in the relation between the maximum width Wmax and the diameter R1 of the inlet port 54, and preferably satisfies the condition of the following inequality (2) in the relation between the maximum width Wmax and the diameter R2 of the outlet port 55.

$$R1 \times 0.9 < Wmax < R1 \times 1.1 \qquad (1)$$

$$R2 \times 0.9 < Wmax < R2 \times 1.1 \qquad (2)$$

The distance in the height direction h between the lowest position 56B of the addition tank internal channel 56 and the lowest position 54B of the inlet port 54 is preferably less than 0.1 times the diameter R1 of the inlet port 54 and more preferably 0. The distance in the height direction h between the lowest position 56B of the addition tank internal channel 56 and the lowest position 55B of the outlet port 55 is preferably less than 0.1 times the diameter R2 of the outlet port 55 and more preferably 0.

As shown in Fig. 5, the addition tank 33 has sloping surfaces 57 which extend from upper ends 56A of the addition tank internal channel 56 and slope upward in directions away from the addition tank internal channel 56 (widthwise directions of the addition tank internal channel 56).

The addition tank 33 has an introduction portion 60 and a ventilation portion 70.

The introduction portion 60 is a portion through which a liquid different from the measurement liquid S is introduced into the internal space 50 from the outside of the addition tank 33. The supply section 40 is connected to the introduction portion 60, and the liquid is supplied from the supply section 40. The liquid supplied from the supply section 40 is introduced into the internal space 50 through the introduction portion 60. The introduction portion 60 is disposed on a wall surface of the addition tank 33 that faces the internal space 50 above the addition tank internal channel 56. Namely, the lower end of the introduction portion 60 is located higher than the upper ends 56A of the addition tank internal channel 56.

As shown in Fig. 2, the introduction portion 60 includes an adjustment liquid introduction portion 61 and cleaning liquid introduction portions 62. The adjustment liquid supply section 41 is connected to the adjustment liquid introduction portion 61, and the adjustment liquid is supplied from the adjustment liquid supply section 41. The adjustment liquid supplied from the adjustment liquid supply section 41 is introduced into the internal space 50 through the adjustment liquid introduction portion 61. The cleaning liquid supply section 42 is connected to the cleaning liquid introduction portions 62, and the cleaning liquid is supplied from the cleaning liquid supply section 42. The cleaning liquid supplied from the cleaning liquid supply section 42 is introduced into the internal space 50 through the cleaning liquid introduction portions 62.

As shown in Figs. 2, 3, and 5, the adjustment liquid introduction portion 61 and the cleaning liquid introduction portions 62 are provided on the top wall 53 of the addition tank 33. The adjustment liquid introduction portion 61 is disposed right above the addition tank internal channel 56. There is provided only one adjustment liquid introduction portion 61. The cleaning liquid introduction portions 62 are provided right above the sloping surfaces 57. The number of the cleaning liquid introduction portions 62 is two. The cleaning liquid introduction portions 62 are provided such that one cleaning liquid introduction portion 62 is provided on each of opposite sides in the widthwise direction of the addition tank internal channel 56.

The ventilation portion 70 is a portion for releasing air from the internal space 50 to the outside. The ventilation portion 70 is disposed on the wall surface of the addition tank 33 that faces the internal space 50 above the addition tank internal channel 56. Namely, the lower end of the ventilation portion 70 is located higher than the upper ends 56A of the addition tank internal channel 56. As shown in Fig. 3, the ventilation portion 70 includes a first ventilation portion 71 and a second ventilation portion 72.

As shown in Fig. 3, the first ventilation portion 71 is provided on the top wall 53 of the addition tank 33. The first ventilation portion 71 has a water repellent filter which prevents entry of liquid into the internal space 50 from the outside. As shown in Fig. 3, the second ventilation portion 72 is provided in the side wall 52 of the addition tank 33. The lower end of the second ventilation portion 72 is located higher than the upper ends 56A of the addition tank internal channel 56, and the upper end of the second ventilation portion 72 is located lower than the lower end of the introduction portion 60.

The sectional area of an opening 70A of the ventilation portion 70 (the sum of the sectional area of an opening 71A of the first ventilation portion 71 and the sectional area of an opening 72A of the second ventilation portion 72) is greater than the sectional area of the inlet port 54 and the sectional area of the outlet port 55.

1-3. Operation of liquid quality measurement apparatus 1

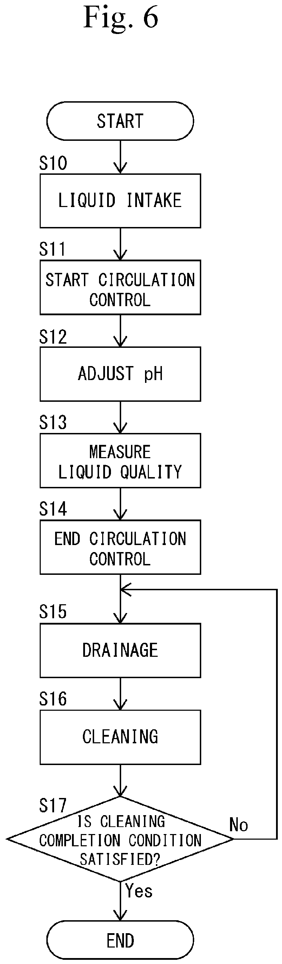

The liquid quality measurement apparatus 1 performs a process illustrated in Fig. 6 when it measures the quality of the measurement liquid S. When an intake start condition is satisfied, in step S10, the liquid quality measurement apparatus 1 performs intake control for taking the measurement liquid S into the circulation path 10. The intake start condition may be, for example, that a predetermined intake start operation is performed by an operator, or another condition. In the intake control, the liquid quality measurement apparatus 1 takes the measurement liquid S into the circulation path 10 by switching the three-way valve 20 and the three-way valve 25 to their first states, switching the two-way valve 23 corresponding to the measurement liquid S to be taken to its open state, and activating the pump 30.

When an intake completion condition is satisfied, the liquid quality measurement apparatus 1 ends the intake control. The intake completion condition may be, for example, that the liquid level in the internal space 50 of the addition tank 33 has reached an intake completion position, that the amount of the taken-in liquid reaches a predetermined amount, or another condition. The determination as to whether or not the liquid level has reached the intake completion position may be performed, for example, by providing a liquid level sensor which detects the liquid level in the internal space 50 and performing the determination on the basis of the liquid level detected by the liquid level sensor. Alternatively, the determination as to whether or not the liquid level has reached the intake completion position may be performed by providing a flow rate sensor in the circulation path 10 or the common path 21 and performing the determination on the basis of the flow rate detected by that flow rate sensor. The determination as to whether or not the amount of the taken-in liquid has reached the predetermined amount may be performed, for example, by providing a flow rate sensor in the circulation path 10 or the common path 21 and performing the determination on the basis of the flow rate detected by that flow rate sensor or by another method.

As a result of the intake control being performed in this manner, the measurement liquid S is charged into the circulation path 10. The liquid level in the internal space 50 becomes, for example, higher than the upper ends 56A of the addition tank internal channel 56 and lower than the lower end of the introduction portion 60.

After having ended the intake control, in step S11, the liquid quality measurement apparatus 1 starts circulation control. In the circulation control, the liquid quality measurement apparatus 1 switches the three-way valve 20 and the three-way valve 25 to their second states and activates the pump 30. As a result, the measurement liquid S circulates within the circulation path 10.

In step S12, the liquid quality measurement apparatus 1 adjusts the pH of the measurement liquid S within the circulation path 10. In the liquid quality adjustment, the liquid quality measurement apparatus 1 causes, on the basis of the Ph detected by the pH sensor 32, the adjustment liquid supply section 41 to supply the pH adjustment liquid until the liquid quality measurement apparatus 1 determines that the pH of the measurement liquid S has exceeded a predetermined threshold value. When the liquid quality measurement apparatus 1 determines that the pH of the measurement liquid S has exceeded the threshold value, the liquid quality measurement apparatus 1 ends the liquid quality adjustment.

After having completed the liquid quality adjustment, in step S13, the liquid quality measurement apparatus 1 starts liquid quality measurement operation by the liquid quality sensor 31. The liquid quality measurement apparatus 1 adopts a measurement value obtained by this liquid quality measurement operation as the liquid quality value of the measurement liquid S.

After having completed the liquid quality measurement by the liquid quality sensor 31, in step S14, the liquid quality measurement apparatus 1 ends the circulation control started in step S11. After having ended the circulation control, in step S15, the liquid quality measurement apparatus 1 performs drainage control for draining the measurement liquid S within the circulation path 10. In the drainage control, the liquid quality measurement apparatus 1 switches the three-way valve 20 and the three-way valve 25 to their third states and activates the pump 30. As a result, the measurement liquid S within the circulation path 10 is drained through the drain passage 26. When a drainage completion condition is satisfied, the liquid quality measurement apparatus 1 ends the drainage control. The drainage completion condition may be, for example, that a predetermined drainage time has elapsed after start of the drainage control, or another condition.

After having ended the drainage control, in step S16, the liquid quality measurement apparatus 1 performs cleaning control for cleaning the interior of the circulation path 10 by using the cleaning liquid. In the cleaning control, the liquid quality measurement apparatus 1 switches the three-way valve 20 and the three-way valve 25 to their second states, causes the cleaning liquid supply section 42 to supply the cleaning liquid, and activates the pump 30. When a predetermined cleaning time has elapsed after the cleaning control had been started, in step S17, the liquid quality measurement apparatus 1 determines whether or not a cleaning completion condition is satisfied. The liquid quality measurement apparatus 1 determines whether or not the cleaning completion condition is satisfied, for example, on the basis of the pH of the liquid within the circulation path 10. The liquid quality measurement apparatus 1 determines that the cleaning completion condition is satisfied, for example, in the case where the pH of the liquid within the circulation path 10 falls within a predetermined range set beforehand.

In the case where the liquid quality measurement apparatus 1 determines that the cleaning completion condition is not satisfied (the case where the result of the determination in step S17 is "No"), the liquid quality measurement apparatus 1 returns to step S15. Namely, the liquid quality measurement apparatus 1 repeats the drainage control and the cleaning control until the liquid quality measurement apparatus 1 determines that the cleaning completion condition is satisfied. In the case where the liquid quality measurement apparatus 1 determines that the cleaning completion condition is satisfied (the case where the result of the determination in step S17 is "Yes"), the liquid quality measurement apparatus 1 switches the three-way valve 20 and the three-way valve 25 to their third states, thereby draining the cleaning liquid, and ends the process of Fig. 6.

1-4. Effects of the first embodiment

The liquid quality measurement apparatus 1 of the first embodiment includes the addition tank 33 which constitutes a portion of the circulation path 10, and the introduction portion 60 and the ventilation portion 70 are provided in the addition tank 33. Therefore, it is possible to appropriately add a liquid different from the measurement liquid S to the internal space 50 of the addition tank 33 through the introduction portion 60 while releasing air from the internal space 50 of the addition tank 33 through the ventilation portion 70.

Furthermore, the maximum width Wmax of the addition tank internal channel 56 satisfies the condition of the following inequality (1) in the relation between the maximum width Wmax and the diameter R1 of the inlet port 54, and satisfies the condition of the following inequality (2) in the relation between the maximum width Wmax and the diameter R2 of the outlet port 55.

$$R1x0.9 < Wmax < R1x1.1 \qquad (1)$$

$$R2x0.9 < Wmax < R2x1.1 \qquad (2)$$

Therefore, the amount of the measurement liquid S necessary to be charged into the circulation path 10 can be reduced.

Furthermore, in the addition tank 33, the distance in the height direction h between the lowest position 56B of the addition tank internal channel 56 and the lowest position 54B of the inlet port 54 is less than 0.1 times the diameter R1 of the inlet port 54, and the distance in the height direction h between the lowest position 56B of the addition tank internal channel 56 and the lowest position 55B of the outlet port 55 is less than 0.1 times the diameter R2 of the outlet port 55. Therefore, when the measurement liquid S within the circulation path 10 is drained, it is possible to prevent the measurement liquid S from remaining at the bottom of the addition tank internal channel 56.

Particularly, in the present embodiment, in the addition tank 33, the distance in the height direction h between the lowest position 56B of the addition tank internal channel 56 and the lowest position 54B of the inlet port 54 is 0, and the distance in the height direction h between the lowest position 56B of the addition tank internal channel 56 and the lowest position 55B of the outlet port 55 is 0. Therefore, when the measurement liquid S within the circulation path 10 is drained, it is possible to more reliably prevent the measurement liquid S from remaining at the bottom of the addition tank internal channel 56.

Furthermore, the upper ends 56A of the addition tank internal channel 56 are located higher than the upper end 54A of the inlet port 54 and the upper end 55A of the outlet port 55. Therefore, the amount of the measurement liquid S necessary to be charged into the circulation path 10 can be reduced.

Furthermore, the addition tank 33 has the sloping surfaces 57 which extend from upper ends 56A of the addition tank internal channel 56 and slope upward in directions away from the addition tank internal channel 56. Therefore, the measurement liquid S having accumulated in the internal space 50 of the addition tank 33 is guided to the addition tank internal channel 56 by the sloping surface 57, and as a result, the measurement liquid S is easily drained from the addition tank internal channel 56.

Furthermore, the adjustment liquid introduction portion 61 is disposed right above the addition tank internal channel 56. Therefore, it is possible to prevent the adjustment liquid introduced from the introduction portion 60 from being uselessly supplied to a location outside the addition tank internal channel 56.

Furthermore, the introduction portion 60 is provided on the top wall 53 of the addition tank 33. Therefore, flow of the measurement liquid S into the introduction portion 60 can be easily prevented.

Furthermore, the sectional area of the opening 70A of the ventilation portion 70 (the sum of the sectional area of the opening 71A of the first ventilation portion 71 and the sectional area of the opening 72A of the second ventilation portion 72) is greater than the sectional area of the inlet port 54 and the sectional area of the outlet port 55. Therefore, an amount of air that is equal to or greater than the amount of the measurement liquid S supplied to the internal space 50 can be easily released from the ventilation portion 70. Accordingly, flow of the measurement liquid S into the introduction portion 60 can be easily prevented.

Furthermore, the ventilation portion 70 is provided in the side wall 52 of the addition tank 33. Therefore, when the level of the measurement liquid S exceeds a predetermined level in the internal space 50 of the addition tank 33, an excessive portion of the measurement liquid S can be drained to the outside.

The liquid quality measurement system 100 of the first embodiment includes the liquid quality measurement apparatus 1, the adjustment liquid supply section 41, and the monitoring apparatus 49. The adjustment liquid supply section 41 supplies the adjustment liquid through the introduction portion 60 under the control by the liquid quality measurement apparatus 1. The monitoring apparatus 49 monitors the quality of the measurement liquid S measured by the liquid quality sensor 31. The liquid quality measurement apparatus 1 starts the liquid quality measurement operation by the liquid quality sensor 31 after the liquid quality adjustment performed for the measurement liquid S by using the adjustment liquid has been completed. This liquid quality measurement system 100 makes it possible to obtain the liquid quality value of the measurement liquid S in a state after completion of the liquid quality adjustment.

Furthermore, the liquid quality measurement apparatus 1 further includes the pH sensor 32 for measuring the pH of the measurement liquid S and, as the liquid quality adjustment, the liquid quality measurement apparatus 1 causes the adjustment liquid supply section 41 to supply the adjustment liquid until the liquid quality measurement apparatus 1 determines that the pH has exceeded a threshold value. Therefore, the liquid quality measurement system 100 makes it possible to obtain the liquid quality value of the measurement liquid S whose pH has been adjusted.

<Other embodiments>

The present invention is not limited to the embodiment described by the above description and the drawings, and, for example, the following embodiments fall within the technical scope of the present invention. Also, various features of the above-described embodiment and the following embodiments can be combined freely so long as no contradiction occurs.

In the above-described embodiment, the liquid quality measurement system is configured such that it is mounted on a liquid piping line of a biogas plant. However, the liquid quality measurement system may be configured differently. The liquid quality measurement system may be configured to be mounted on a liquid piping line of a land-based aquaculture system, a methane fermentation tank, a sewage disposal plant, or a facility where management of waste liquid such as industrial waste water is necessary.

In the above-described embodiment, the first ventilation portion has a water repellent filter. However, the water repellent filter may be omitted.

In the above-described embodiment, the liquid quality measurement apparatus has both the first ventilation portion and the second ventilation portion. However, the liquid quality measurement apparatus may be configured to have at least one of the first ventilation portion and the second ventilation portion.

In the above-described embodiment, the liquid quality measurement apparatus is configured to start the liquid measurement operation by the liquid quality sensor after the liquid adjustment performed for the measurement liquid by using the adjustment liquid has been completed. However, the liquid quality measurement apparatus may be configured differently. For example, the liquid quality measurement apparatus may be configured to execute the liquid adjustment performed for the measurement liquid by using the adjustment liquid and the liquid measurement operation by the liquid quality sensor in parallel, and may adopt the measurement value measured by the liquid quality sensor after completion of the liquid quality adjustment as the liquid quality value of the measurement liquid.

Notably, the embodiments disclosed this time should be considered to be illustrative and not to be restrictive in all aspects. The scope of the present invention is not limited to the embodiments disclosed this time, and it is intended that the present invention encompasses all modifications within the range shown by the claims and the range of equivalents of the claims.

Although the preferred embodiments of the present invention have been described above, the liquid quality measurement apparatus and the liquid quality measurement system according to the present invention can be used for various purposes. For example, the present invention can be applied in fields in which water quality must be measured, such as offshore aquaculture, land-based aquaculture, water treatment, waste water treatment, aquarium, agriculture (hydroponics).

Description of Reference Numerals and Symbols

1: liquid quality measurement apparatus
10: circulation path
31: liquid quality sensor
32: pH sensor
33: addition tank
40: supply section
41: adjustment liquid supply section
49: monitoring apparatus
50: internal space
50A: bottom portion
52: side wall
53: top wall
54: inlet port
54A: upper end of the inlet port
54B: lowest position of the inlet port
55: outlet port
55A: upper end of the outlet port
55B: lowest position of the outlet port
56: addition tank internal channel
56A: upper end of the addition tank internal channel
56B: lowest position of the addition tank internal channel
57: sloping surface
60: introduction portion
61: adjustment liquid introduction portion
62: cleaning liquid introduction portion
70: ventilation portion
70A: opening of the ventilation portion
71: first ventilation portion
71A: opening of the first ventilation portion
72: second ventilation portion
72A: opening of the second ventilation portion
90: piping
100: liquid quality measurement system
H1: height of the center of the inlet port from the lowest position of the addition tank internal channel
H2: height of the center of the outlet port from the lowest position of the addition tank internal channel
S: measurement liquid
Wmax: maximum width of the addition tank internal channel

**Claims**

1. A liquid quality measurement apparatus (1) comprising a piping (90) that constitutes a circulation path (10) through which a measurement liquid flows, and a liquid quality sensor (31) for measuring a quality of the measurement liquid within the circulation path (10),

    the liquid quality measurement apparatus (1) further comprising an addition tank (33) that is connected to the piping (90) via an inlet port (54) and an outlet port (55), constitutes a portion of the circulation path (10), and has an internal space (50), wherein
    the addition tank (33) has an introduction portion (70) through which a liquid different from the measurement liquid is introduced into the internal space (50) from outside of the addition tank (33), and a ventilation portion (60) through which air is released from the internal space (50) to the outside;
    an addition tank internal channel (56) is formed at a bottom portion of the internal space (50), the addition tank internal channel (56) connecting the inlet port (54) and the outlet port (55) and allowing the measurement liquid to flow through the addition tank internal channel (56); and
    the introduction portion (70) and the ventilation portion (60) are disposed in a wall surface of the addition tank (33) that faces the internal space (50) above the addition tank internal channel (56).

2. A liquid quality measurement apparatus (1) according to claim 1, wherein a maximum width Wmax of the addition tank internal channel (56) satisfies a condition of the following inequality (1) in a relation between the maximum width Wmax and a diameter R1 of the inlet port (54), and satisfies a condition of the following inequality (2) in a relation between the maximum width Wmax and a diameter R2 of the outlet port (55).

$$R1 \times 0.9 < Wmax < R1 \times 1.1 \qquad (1)$$

$$R2 \times 0.9 < Wmax < R2 \times 1.1 \qquad (2)$$

3. A liquid quality measurement apparatus (1) according to claim 1, wherein, in the addition tank (33), a distance in a height direction between a lowest position of the addition tank internal channel (56) and a lowest position of the inlet port (54) is less than 0.1 times the diameter R1 of the inlet port (54), and a distance in the height direction between the lowest position of the addition tank internal channel (56) and a lowest position of the outlet port (55) is less than 0.1 times the diameter R2 of the outlet port (55).

4. A liquid quality measurement apparatus (1) according to any one of claims 1 to 3, wherein an upper end of the addition tank internal channel (56) is located higher than an upper end of the inlet port (54) and an upper end of the outlet port (55).

5. A liquid quality measurement apparatus (1) according to claim 4, wherein the addition tank (33) has a sloping surface (57) which extends from the upper end of the addition tank internal channel (56) and slopes upward in a direction away from the addition tank internal channel (56).

6. A liquid quality measurement apparatus (1) according to any one of claims 2 to 5, wherein the introduction portion (70) for introducing the different liquid is disposed right above the addition tank internal channel (56).

7. A liquid quality measurement apparatus (1) according to any one of claims 1 to 6, wherein the introduction portion (70) is provided on a top wall of the addition tank (33).

8. A liquid quality measurement apparatus (1) according to any one of claims 1 to 7, wherein an opening of the ventilation portion (60) has a sectional area greater than a sectional area of the inlet port (54) and a sectional area of the outlet port (55).

9. A liquid quality measurement apparatus (1) according to any one of claims 1 to 8, wherein the ventilation portion (60) is provided in a side wall of the addition tank (33).

10. A liquid quality measurement system comprising:

a liquid quality measurement apparatus (1) according to any one of claims 1 to 9;
an adjustment liquid supply section (41) that supplies an adjustment liquid, as the different liquid, through the introduction portion (70) under control performed by the liquid quality measurement apparatus (1); and
a monitoring apparatus (49) that monitors the quality of the measurement liquid measured by the liquid quality sensor (31), wherein the liquid quality measurement apparatus (1) starts liquid quality measurement operation by the liquid quality sensor (31) after liquid quality adjustment performed for the measurement liquid by using the adjustment liquid has been completed, or adopts a measurement value measured by the liquid quality sensor (31) after completion of the liquid quality adjustment as a liquid quality value of the measurement liquid.

11. A liquid quality measurement system according to claim 10, wherein the liquid quality measurement apparatus (1) further comprises a pH sensor for measuring pH of the measurement liquid, and, as the liquid quality adjustment, the liquid quality measurement apparatus (1) causes the adjustment liquid supply section to supply the adjustment liquid until the liquid quality measurement apparatus (1) determines that the pH has exceeded a threshold value.

# Fig. 1

# Fig. 2

# Fig. 3

# Fig. 4

# Fig. 5

# Fig. 6

```
                    ┌─────────────┐
                    │    START    │
                    └──────┬──────┘
   S10                     │
        ┌──────────────────▼──────────────────┐
        │           LIQUID INTAKE             │
        └──────────────────┬──────────────────┘
   S11                     │
        ┌──────────────────▼──────────────────┐
        │        START CIRCULATION            │
        │            CONTROL                  │
        └──────────────────┬──────────────────┘
   S12                     │
        ┌──────────────────▼──────────────────┐
        │            ADJUST pH                │
        └──────────────────┬──────────────────┘
   S13                     │
        ┌──────────────────▼──────────────────┐
        │            MEASURE                  │
        │         LIQUID QUALITY              │
        └──────────────────┬──────────────────┘
   S14                     │
        ┌──────────────────▼──────────────────┐
        │        END CIRCULATION             │
        │            CONTROL                  │
        └──────────────────┬──────────────────┘
                           │
   S15                     ▼
        ┌──────────────────────────────────────┐
        │            DRAINAGE                 │
        └──────────────────┬──────────────────┘
   S16                     │
        ┌──────────────────▼──────────────────┐
        │            CLEANING                 │
        └──────────────────┬──────────────────┘
                           │
   S17                     ▼
              ◇ IS CLEANING              ◇  No
         COMPLETION CONDITION
              SATISFIED?
                     │ Yes
                     ▼
              ┌─────────────┐
              │     END     │
              └─────────────┘
```

Europäisches
Patentamt

European
Patent Office

Office européen
des brevets

# EUROPEAN SEARCH REPORT

Application Number

EP 22 17 6417

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/099434 A1 (BIOSAFE SYSTEMS LLC) 23 May 2019 (2019-05-23) <br> * page 1, lines 28-30 * <br> * page 5, lines 4-13 * <br> * page 5, lines 17-19 * <br> * page 6, lines 7-9 * <br> * figure 1 * <br> ----- | 1-8,10, 11 | INV. <br> G01N1/20 <br> G01N33/18 <br><br> ADD. <br> G01N1/10 |
| X | US 2021/146367 A1 (SZPAK JAMES EDWARD [US] ET AL) 20 May 2021 (2021-05-20) <br> * paragraph [0215] * <br> * paragraph [0255] * <br> * paragraph [0257] * <br> * figure 16 * <br> ----- | 1-3,5,8, 9 | |
| A | US 2020/340968 A1 (ZAKINOV NELSON [IL]) 29 October 2020 (2020-10-29) <br> * paragraph [0043] * <br> * paragraph [0087] * <br> * figure 3 * <br> ----- | 1-11 | |
| | | | TECHNICAL FIELDS SEARCHED (IPC) <br><br> G01N |

The present search report has been drawn up for all claims

| Place of search | Date of completion of the search | Examiner |
|---|---|---|
| The Hague | 30 September 2022 | Traversa, Marzia |

EPO FORM 1503 03.82 (P04C01)

## ANNEX TO THE EUROPEAN SEARCH REPORT
## ON EUROPEAN PATENT APPLICATION NO.

EP 22 17 6417

This annex lists the patent family members relating to the patent documents cited in the above-mentioned European search report.
The members are as contained in the European Patent Office EDP file on
The European Patent Office is in no way liable for these particulars which are merely given for the purpose of information.

30-09-2022

| Patent document cited in search report | | Publication date | Patent family member(s) | | Publication date |
|---|---|---|---|---|---|
| WO 2019099434 | A1 | 23-05-2019 | CA | 3081360 A1 | 23-05-2019 |
| | | | US | 2021179460 A1 | 17-06-2021 |
| | | | US | 2022081335 A1 | 17-03-2022 |
| | | | WO | 2019099434 A1 | 23-05-2019 |
| US 2021146367 | A1 | 20-05-2021 | US | 2021146367 A1 | 20-05-2021 |
| | | | WO | 2021097188 A1 | 20-05-2021 |
| US 2020340968 | A1 | 29-10-2020 | GB | 2561838 A | 31-10-2018 |
| | | | US | 2020340968 A1 | 29-10-2020 |
| | | | WO | 2018198018 A1 | 01-11-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012083293 A